Europäisches Patentamt

European Patent Office

Office européen des brevets

(11) Veröffentlichungsnummer: **0 252 900**
**A2**

## (12) EUROPÄISCHE PATENTANMELDUNG

(21) Anmeldenummer: **87890144.6**

(22) Anmeldetag: **29.06.87**

(51) Int. Cl.⁴: **C 12 N 1/08**
**//(C12N1/08,C12R1:72,1:865)**

(30) Priorität: **03.07.86 AT 1804/86**

(43) Veröffentlichungstag der Anmeldung:
**13.01.88 Patentblatt 88/02**

(84) Benannte Vertragsstaaten:
**BE CH DE FR GB IT LI NL**

(71) Anmelder: **Vogelbusch Gesellschaft m.b.H.**
**Blechturmgasse 11**
**A-1050 Wien (AT)**

(72) Erfinder: **Gibert, Eric, Dr.**
**Haiden 361**
**A-5351 Aigen-Voglhub (AT)**

(74) Vertreter: **Wolfram, Gustav, Dipl.-Ing.**
**Schwindgasse 7 P.O. Box 205**
**A-1041 Wien (AT)**

(54) **Verfahren zur Gewinnung von proteinhältigen Mikroorganismenzellen.**

(57) Bei der Gewinnung eines einzelligen Protein-Materials (single cell protein, SCP) wird, um in kurzer Zeit ohne beträchtliche Proteinverluste und ohne signifikante Zellzerstörung mit einfacher apparativer Ausrüstung und unter Einsatz billiger Chemikalien eine Verringerung des RNS-Gehaltes im gewonnenen Zellmaterial auf höchstens 1,5 bis 2 Massen% zu erzielen,
- die Zellensuspension mit einer Mineralsäure bis zu einer Säurekonzentration von 0,1 bis 1,0 M, vorzugsweise 0,2 bis 0,4 M versetzt und während eines Zeitraumes bis 20 min bei Raumtemperatur durchgemischt,
- anschließend die angesäuerte Zellensuspension 3 bis 10 min, vorzugsweise 6 bis 9 min lang auf 60 bis 95°C, vorzugsweise 70 bis 90°C, erwärmt,
- die wärmebehandelte Zellensuspension abgekühlt, mit Alkali auf einen pH-Wert von 5,5 bis 8,5 eingestellt und
- die Nucleinsäureabbauprodukte enthaltende wässerige Phase von der Zellenmasse abgetrennt und letztere in eine haltbare Form gebracht.

EP 0 252 900 A2

0 252 900

**Beschreibung**

Verfahren zur Gewinnung von proteinhältigen Mikroorganismenzellen

Die Erfindung betrifft ein Verfahren zur Gewinnung von proteinhältigen Mikroorganismenzellen mit verringertem Nucleinsäuregehalt durch Behandlung einer wässerigen Zellensuspension mit Mineralsäure unter Erwärmung, sowie anschließende Behandlung mit Alkali in wässerigem Medium.

Einem solchen einzelligen Protein-Material (single cell protein, SCP) kommt angesichts der Ernährungsprobleme der schnellwachsenden Weltbevölkerung als neue Proteinquelle größte Bedeutung zu. Da Mikroorganismen sehr schnell wachsen und verschiedenste Rohstoffquellen nützen können, rücken sie immer mehr in den Brennpunkt des Interesses.

Während der Wachstumsphase bauen die Zellen die für ihre Existenz notwendigen Proteine auf. Bei der Gewinnung von SCP als Eiweißlieferant liegt das Hauptinteresse auf der Gewinnung der ganzen Zellen. Daraus folgt, daß auf die einzelnen Bestandteile der Zelle, also die Proteine, Polysaccharide, Lipide und Nucleinsäuren ganz besonders Bedacht zu nehmen ist, da diesen sehr unterschiedliche Bedeutung zukommt.

Seit etwa 30 Jahren kennt man den Zusammenhang zwischen Harnsäure-Blutspiegel und Nucleinsäure-Stoffwechsel des Menschen sowie dem Auftreten von Gichterkrankungen. Auch ohne besondere Veranlagung dazu kann diese Krankheit bei Verzehr purinreicher Kost (Purine sind die Bestandteile von Ribonucleinsäure, RNS) über längere Zeit manifest werden. Der begrenzende Faktor für den Einsatz von SCP in der menschlichen Ernährung liegt in dessen relativ hohem Gehalt an Nucleinsäuren. Da der Gehalt an RNS in SCP durchschnittlich 5 bis 8 % beträgt (Bakterienzellen sogar 12 bis 18 %), kann SCP ohne weitere Aufbereitung höchstens ein Fünftel der pro Tag notwendigen Eiweißaufnahme ohne gesundheitliche Risken decken.

Die Weltgesundheitsorganisation (WHO) empfiehlt daher eine tägliche Dosis an RNS von nicht mehr als 2 g pro männlichem Erwachsenen mit 65 kg Körpergewicht und 1,7 g pro weiblichem Erwachsenen mit 55 kg Körpergewicht. Das entspricht bei maximal 8 % RNS - beispielsweise in Hefe - ca. 20 bis 25 g Hefetrockensubstanz (HTS)/Tag, ohne daß die Konzentration an Harnsäure zu hohe Werte erreicht. Der tägliche Eiweißbedarf eines Erwachsenen beträgt aber ca. 70 g.

Der Genuß von RNS führt bei den Primaten, bedingt durch das Fehlen eines Enzyms (Uricase), zu einem erhöhten Harnsäurespiegel im Blut, der nicht weiter abgebaut werden kann. Da die Harnsäure aber in wässerigem Milieu niedrige Löslichkeit aufweist, sammelt sie sich im Körper in kristalliner Form an. Dies kann zu Zuständen führen, die als Gicht bekannt sind. Viele Ernährungswissenschaftler empfehlen daher, daß die RNS-Aufnahme möglichst niedrig gehalten wird. In SCP, das für den menschlichen Gebrauch bestimmt ist, sollte der RNS-Gehalt vorzugsweise auf ungefähr 3 %, bezogen auf das Trockengewicht der Zellen, vermindert werden.

Laut P.A.G. (Protein-calorie Advisory Group) müßte es reichen, den Gehalt von SCP an RNS auf ca. 3 % herabzusetzen, um es als hauptsächliche Proteinquelle einzusetzen.

Innerhalb der letzten ca. 15 Jahre wurden eine Vielzahl von Verfahren zur RNS-Reduktion in SCP entwickelt, wobei u.a. Behandlungen mit alkalischen und sauren Agentien, mit organischen Lösungsmitteln, Enzymen, Hitzeschocks angewendet wurden.

Ein Verfahren der eingangs erwähnten Art ist aus der EP-A2 - 0 050 831 bekannt, wonach eine stark verdünnte Suspension einzelligen Protein-Materials direkt aus einer Fermentation einer Behandlung zur Verringerung des Nucleinsäuregehaltes der Mikroorganismen zugeführt wird. Diese RNS-Reduktionsbehandlung kann beispielsweise aus dem Einwirken einer Säure unter erhöhter Temperatur bestehen. Anschließend wird die Suspension in eine Zellmasse und eine nucleinsäurehältige Flüssigkeit aufgetrennt, welch letztere wenigstens teilweise in die Fermentation rückgeführt werden kann. Die abgetrennte Zellmasse mit reduziertem RNS-Gehalt wird sodann bei erhöhter Temperatur mit einer Base bei einem pH-Wert von etwa 10,6 oder höher extrahiert, wobei ein Proteingemisch mit reduziertem Nucleinsäuregehalt in flüssiger und fester Phase anfällt.

Durch Trennung der beiden Phasen wird eine Lösung von Proteinen mit reduziertem Gehalt an Nucleinsäuren als ein erstes Produkt und ein festes Protein-Zellmaterial mit reduziertem Nucleinsäuregehalt als zweites Produkt gewonnen.

Da verdünnte Zellsuspensionen eingesetzt werden und relativ lange Behandlungszeiträume eingehalten werden müssen, ist das bekannte Verfahren wenig wirtschaftlich und mit hohem Energieaufwand verbunden. Zudem kommt es im Verlauf der Extraktion mit alkalischen Medien bei erhöhter Temperatur zur Bildung unerwünschter Substanzen wie Lysinoalaninen. Die teilweise Zerstörung der Zellmembranen führt zu einem Ballaststoffverlust.

In der DE-C - 26 33 666 ist ein Verfahren zur Verminderung des Lipid- und Nucleinsäuregehaltes in mikrobiellen Zellmassen durch Behandlung mit einer Extraktionsmittelmischung aus $NH_3$ oder $NH_4$ OH und einem organischen, Hydroxylgruppen enthaltenden Lösungsmittel bei -20° C bis 60° C beschrieben, wobei der Gesamt-Wassergehalt während der Extraktion 0 bis 30 Gew.%, bezogen auf die eingesetzte Lösungsmittelmenge, beträgt. Der Zell-Rückstand wird mit Wasser bei einem pH-Wert von 5 bis 8,5 behandelt. Dieses Verfahren erfordert unbedingt eine energieaufwendige Trocknungsbehandlung der eingesetzten Zellmassen vor der Extraktion. So ist es beispielsweise nicht möglich, Frischhefe bzw. Heferahm direkt einzusetzen. Außerdem müssen bei diesem bekannten Verfahren aus Gründen der Wirtschaftlichkeit und des

2

Umweltschutzes die organischen Lösungsmittel rückgewonnen werden.

Die Erfindung bezweckt die Überwindung der dargelegten, mit den bekannten Verfahren verbundenen Nachteile und Schwierigkeiten und stellt sich die Aufgabe, in kurzer Zeit ohne beträchtliche Proteinverluste und ohne signifikante Zellzerstörung mit einfacher apparativer Ausrüstung und unter Einsatz billiger Chemikalien eine Verringerung des RNS-Gehaltes im gewonnenen Zellmaterial auf höchstens 1,5 bis 2 Massen% zu erzielen. Eine RNS-Reduktion auf diesen Prozentgehalt qualifiziert einzelliges Protein-Material sogar zur Verwendung als alleinige Eiweißquelle für die menschliche Ernährung.

Die gestellte Aufgabe wird bei einem Verfahren der eingangs definierten Art erfindungsgemäß dadurch gelöst, daß
- die Zellensuspension mit einer Mineralsäure bis zu einer Säurekonzentration von 0,1 bis 1,0 M, vorzugsweise 0,2 bis 0,4 M versetzt und während eines Zeitraumes bis 20 min bei Raumtemperatur durchgemischt wird,
- anschließend die angesäuerte Zellensuspension 3 bis 10 min, vorzugsweise 6 bis 9 min lang auf 60 bis 95°C, vorzugsweise 70 bis 90°C, erwärmt wird,
- die wärmebehandelte Zellensuspension abgekühlt, mit Alkali auf einen pH-Wert von 5,5 bis 8,5 eingestellt wird und
- die Nucleinsäureabbauprodukte enthaltende wässerige Phase von der Zellenmasse abgetrennt und letztere in eine haltbare Form gebracht wird.

Die erfindungsgemäß gewonnenen proteinhältigen Mikroorganismenzellen enthalten in jedem Fall weniger als 2 Massen% Nucleinsäuren und im allgemeinen über 50 Massen% Protein, jeweils bezogen auf die Trockenmasse des Zellmaterials. Der Proteingehalt des Produktes ist somit höher als der Proteingehalt der unbehandelten Mikroorganismenzellen.

Die reinen Proteinverluste sind gering, nämlich unter 10 %. Dieser geringe Proteinverlust ist ein sehr bedeutender Vorteil des erfindungsgemäßen Verfahrens.

Die abgetrennte wässerige Phase kann auf verschiedene Weise weiter aufgearbeitet werden, um die RNS-Fraktion zurückzugewinnen, beispielsweise durch Ausfällen mit Säuren oder durch Separation mittels spezieller Membranen.

Nach einer Ausführungsform des erfindungsgemäßen Verfahrens wird zumindest ein Teil der eingesetzten Mineralsäure durch ein Salz derselben oder einer anderen Säure ersetzt, wobei die Gesamtmolarität der entsprechenden Säureanionen nicht verändert wird. Als Salze der Mineralsäuren werden insbesondere $Na^{\oplus}$-, $K^{\oplus}$- oder $NH_4$ $^{\oplus}$-Salze eingesetzt.

Die Säuren liegen nach dieser Ausführungsform somit zum Teil neutralisiert vor. Bei Verwendung einer derartigen Säure/Salz-Mischung ist die nach Abkühlung zur pH-Einstellung benötigte Laugen-(Alkali-)menge entsprechend reduziert. Das Ausmaß der RNS-Reduktion entspricht im wesentlichen jenem, welches ohne teilweisen Ersatz der Mineralsäure erzielbar ist.

Zweckmäßig wird eine Suspension mit 10 bis 20 Massen%, vorzugsweise 15 bis 18 Massen%, Zellen eingesetzt.

Beispielsweise kann ein Heferahm, wie er von der Separatorenstation kommt, direkt als Ausgangsprodukt verwendet werden. Dabei erübrigt sich die Zugabe weiterer Wassers zur Verdünnung, die Mengen der durch den Prozeß zu führenden Flüssigkeiten und die Abwassermengen werden möglichst gering gehalten.

Vorteilhaft wird eine Suspension von Hefezellen, wie Saccharomyces cerevisiae und Candida utilis, eingesetzt.

Diese und andere, für die menschliche Ernährung zugelassene Hefen sind besonders geeignet, es können jedoch auch Suspensionen anderer Mikroorganismen, wie Bakterien, erfindungsgemäß behandelt werden.

Als Mineralsäure wird vorzugsweise Salz-, Schwefel- oder Phosphorsäure zugesetzt.

Besonders zweckmäßig wird die Temperatur der wärmebehandelten Zellensuspension durch Wärmeaustausch im Gegenstrom zu angesäuerter Zellensuspension rasch gesenkt und die Suspension weiter auf eine Temperatur von 10 bis 15°C, vorzugsweise 13 bis 14°C, abgekühlt.

Der pH-Wert der abgekühlten Suspension wird vorteilhaft auf 6,5 bis 7,5 eingestellt. In diesem Bereich wird ein besonders guter Transport von wasserlöslichen Abbauprodukten der Nucleinsäurefraktion aus den Zellen in die wässerige Phase erreicht.

Die Einstellung des pH-Wertes der abgekühlten Suspension wird insbesondere mit Natrium- oder Kaliumhydroxid vorgenommen.

Die Abtrennung der behandelten Zellenmasse von der überstehenden wässerigen Phase kann beispielsweise durch Filtration oder Zentrifugieren erfolgen, wonach die abgetrennte Zellenmasse zur gründlichen Entfernung anhaftender Nucleinsäurereste vorzugsweise zweimal mit Wasser gewaschen wird.

Wurde von Hefe als Mikroorganismus ausgegangen, ist das gewonnene Produkt von weißer Farbe und unterscheidet sich von der Ausgangshefe geschmacklich nur in der Hinsicht, daß der typische Hefegeschmack geringfügig reduziert ist.

Durch die folgenden Beispiele wird die Erfindung noch näher erläutert. Alle Prozentangaben beziehen sich auf Trockenmasse. Alle Versuche wurden in einem Gegenstromwärmetauscher mit Heißhaltestrecke durchgeführt.

Beispiel 1:

5 g Trockenhefe Saccharomyces cerevisiae wurden in Wasser unter Zusatz von Schwefelsäure suspendiert, um eine Zellenkonzentration von 15 Massen% und eine Säurekonzentration von 0,3 M zu erreichen. Die Suspension wurde 5 min lang bei 20°C Raumtemperatur intensiv gemischt und rasch auf 80°C erhitzt. Diese Temperatur wurde 8 min lang konstant gehalten. Nach rascher Abkühlung auf 13 bis 14°C wurde die Suspension mit Natriumhydroxid neutralisiert (pH = 6,9 bis 7,0). Die Zellenmasse wurde durch Zentrifugieren abgetrennt, mit Wasser zweimal gewaschen und getrocknet. Die Zellenausbeute betrug 83%. Der RNS-Gehalt wurde von 6,2 auf 0,47 % heruntergesetzt. Der korrigierte Proteinverlust [(NRohprotein - NRNS) 6,25] lag bei 11 %, was im Vergleich zu den Verlusten bei bekannten Verfahren als sehr gering zu bezeichnen ist.

Beispiel 2:

15 g Feuchthefe Saccharomyces cerevisiae wurden analog zu Beispiel 1 behandelt (15 massen%ige Suspension, jedoch 0,1 M $H_2 SO_4$ und 0,2 M NaCl, Erhitzen auf 80°C während 8 min).

Zellausbeute 85 %; RNS-Gehalt von 6,1 % auf 1,0 % reduziert, korrigierter Protein-Verlust: 6 %.

Beispiel 3:

Jeweils 100 l 15 %ige Saccharomyces cerevisiae-Zellensuspension wurden bei variierenden Bedingungen hinsichtlich Säurekonzentration und Temperatur behandelt. Als Säure wurde Salzsäure eingesetzt, die Erwärmung erfolgte 7 min lang.

Die Ergebnisse sind in der folgenden Tabelle zusammengefaßt:

| Ausgangs-Hefe | Temperatur $[°C]$ | Säurekonzentration $[M]$ | Zellen-Ausbeute $[\%]$ | Protein-verlust $[\%]$ | RNS-Gehalt der behandelten Zellenmasse $[\%]$ |
|---|---|---|---|---|---|
| Frischhefe | 80 | 0,2 | 85 | 5,5 | 3,4 |
| 1 Saccharo- | 90 | 0,2 | 84 | 11,8 | 3,1 |
| myces cere- | 70 | 0,3 | 87 | 11,2 | 3,3 |
| visiae, Kon- | 80 | 0,3 | 87 | 6,2 | 1,02 |
| trolle, mit | 80 | 0,4 | 85 | 14,6 | 0,32 |
| 6,12 % RNS | | | | | |
| Stellhefe | 75 | 0,3 | 87 | 7,3 | 3,7 |
| 2 Saccharo- | 80 | 0,3 | 81 | 10 | 2,08 |
| myces cere- | 70 | 0,4 | 85 | 8 | 1,68 |
| visiae, Kon- | 75 | 0,4 | 86 | 8,4 | 1,24 |
| trolle, mit | 80 | 0,4 | 84 | 8,6 | 0,1 |
| 8,4 % RNS | | | | | |

Beispiel 4:

Es wird eine 15 %ige Suspension der Frischhefe 1, Saccharomyces cerevisiae, mit einem Gehalt von 6,12 % RNS eingesetzt, nach Zusatz von Salzsäure und Natriumchlorid bis zu einer Konzentration der Suspension von 0,1 M HCl und 0,2 M NaCl wird bei etwa 20°C 5 min lang gerührt, die Temperatur sodann auf 80°C gesteigert und nach einer Verweilzeit von 5,5 min wird die Suspension rasch auf etwa 14°C abgekühlt. Der pH-Wert der abgekühlten Suspension wird durch Zugabe einer wässerigen NaOH-Lösung auf etwa 7 eingestellt.

Zellausbeute: 84 %

Proteinverlust:   10 %
RNS-Gehalt der abgetrennten Zellenmasse: 1,5 %.

Beispiel 5:
   15 %ige Suspension, Dauer der Wärmebehandlung 3,5 min, Säure: $H_2SO_4$

| Hefe | Tempe-ratur $[°C]$ | Säure-konzen-tration $[M]$ | Zellen-Ausbeute $[\%]$ | Protein-verlust $[\%]$ | RNS-Gehalt der behan-delten Zel-lenmasse $[\%]$ |
|---|---|---|---|---|---|
| Saccharo-myces cere-visiae, Kon-trolle, mit 6,6 % RNS | 70 | 0,3 | 83 | 9 | 5,5 |
| | 80 | 0,3 | 81 | 12 | 3,5 |
| | 90 | 0,3* | 60 | 26 | 0,7 |
| | 90 | 0,5 | 60 | 28 | 0,2 |

*) Mischung $H_2SO_4$ + $Na_2SO_4$, wobei 0,15 M $H_2SO_4$ und 0,15 M $Na_2SO_4$ verwendet wurden.

Es ist ersichtlich, daß bei einer Temperatur von 90°C trotz kurzer Behandlungszeit zwar schon vergleichsweise hohe Zellenausbeute- und Proteinverluste auftreten, die RNS-Reduktion jedoch optimal ist.

Beispiel 6:
   5 g Trockenhefe Candida utilis wurden in Wasser unter $H_2SO_4$-Zusatz suspendiert (15 %ige Suspension und 0,3 M $H_2SO_4$). Behandlungstemperatur: 80°C
Behandlungszeit:   8 min
   Nach der Wärmebehandlung wurde die Suspension rasch abgekühlt und mit einer wässerigen Lösung von 100 g NaOH/l auf einen pH-Wert von 7 eingestellt. Die Zellen wurden dann abzentrifugiert, zweimal mit Wasser gewaschen und getrocknet.

| Hefe | Zell-ausbeute [%] | Protein-verlust [%] | RNS-Gehalt [%] | RNS-Gehalt nach erfindungsgemäßer Reduktion [%] |
|---|---|---|---|---|
| Candida utilis TMC Inc. | 74,3 | 15 | 8,41 | 0,55 |
| Candida utilis aus Rüben-melasse | 79,1 | 15 | 4,60 | 0,37 |

Beispiel 7:
20 g Frischhefe Candida utilis wurden in Wasser unter Zusatz von Salzsäure suspendiert (15 %ige Suspension, 0,3 M HCl).
Behandlungstemperatur: 80°C
Behandlungszeit:   9 min
Weiterverarbeitung analog zu Beispiel 6

| Hefe | Zell-ausbeute [%] | Protein-verlust [%] | RNS-Gehalt [%] | RNS-Gehalt nach erfindungsgemäßer Reduktion [%] |
|---|---|---|---|---|
| Candida utilis | 74,5 | 11,7 | 8,86 | 0,67 |

Beispiel 8:
5 g Pruteen-Extrakt wurden auf die gleiche Weise, wie in Beispiel 6 beschrieben, behandelt.

0 252 900

| | Zell-ausbeute [%] | Protein-verlust [%] | RNS-Gehalt [%] | RNS-Gehalt nach erfindungsgemäßer Reduktion [%] |
|---|---|---|---|---|
| Pruteen | 78 | 13,5 | 12,4 | 1,52 |

**Patentansprüche**

1. Verfahren zur Gewinnung von proteinhältigen Mikroorganismenzellen mit verringertem Nucleinsäuregehalt durch Behandlung einer wässerigen Zellensuspension mit Mineralsäure unter Erwärmung, sowie anschließende Behandlung mit Alkali in wässerigem Medium, dadurch gekennzeichnet, daß
- die Zellensuspension mit einer Mineralsäure bis zu einer Säurekonzentration von 0,1 bis 1,0 M, vorzugsweise 0,2 bis 0,4 M versetzt und während eines Zeitraumes bis 20 min bei Raumtemperatur durchgemischt wird,
- anschließend die angesäuerte Zellensuspension 3 bis 10 min, vorzugsweise 6 bis 9 min lang auf 60 bis 95°C, vorzugsweise 70 bis 90°C, erwärmt wird,
- die wärmebehandelte Zellensuspension abgekühlt, mit Alkali auf einen pH-Wert von 5,5 bis 8,5 eingestellt wird und
- die Nucleinsäureabbauprodukte enthaltende wässerige Phase von der Zellenmasse abgetrennt und letztere in eine haltbare Form gebracht wird.
2. Verfahren nach Anspruch 1, dadurch gekennzeichnet, daß zumindest ein Teil der eingesetzten Mineralsäure durch ein Salz derselben oder einer anderen Säure ersetzt wird, wobei die Gesamtmolarität der entsprechenden Säureanionen nicht verändert wird.
3. Verfahren nach Anspruch 2, dadurch gekennzeichnet, daß als Salze der Mineralsäuren Na$^{\oplus}$-, K$^{\oplus}$- und NH$_4$ $^{\oplus}$- Salze eingesetzt werden.
4. Verfahren nach wenigstens einem der Ansprüche 1 bis 3, dadurch gekennzeichnet, daß eine Suspension mit 10 bis 20 Massen%, vorzugsweise 15 bis 18 Massen%, Zellen eingesetzt wird.
5. Verfahren nach wenigstens einem der Ansprüche 1 bis 4, dadurch gekennzeichnet, daß eine Suspension von Hefezellen, wie Saccharomyces cerevisiae und Candida utilis, eingesetzt wird.
6. Verfahren nach wenigstens einem der Ansprüche 1 bis 5, dadurch gekennzeichnet, daß Salz-, Schwefel- oder Phosphorsäure und/oder ein Salz dieser Säuren zugesetzt wird.
7. Verfahren nach wenigstens einem der Ansprüche 1 bis 6, dadurch gekennzeichnet, daß die Temperatur der wärmebehandelten Zellensuspension durch Wärmeaustausch im Gegenstrom zu angesäuerter Zellensuspension rasch gesenkt und die Suspension weiter auf eine Temperatur von 10 bis 15°C, vorzugsweise 13 bis 14°C, abgekühlt wird.
8. Verfahren nach wenigstens einem der Ansprüche 1 bis 7, dadurch gekennzeichnet, daß der pH-Wert der abgekühlten Suspension auf 6,5 bis 7,5 eingestellt wird.
9. Verfahren nach wenigstens einem der Ansprüche 1 bis 8, dadurch gekennzeichnet, daß die Einstellung des pH-Wertes der abgekühlten Suspension mit Natrium- oder Kaliumhydroxid vorgenommen wird.
10. Verfahren nach wenigstens einem der Ansprüche 1 bis 9, dadurch gekennzeichnet, daß die von der wässerigen Phase abgetrennte Zellenmasse zweimal mit Wasser gewaschen wird.

7